Europäisches Patentamt

European Patent Office

Office européen des brevets

(11) Publication number: **0 149 905**
**A2**

# (12) EUROPEAN PATENT APPLICATION

(21) Application number: 84308734.7

(22) Date of filing: 14.12.84

(51) Int. Cl.⁴: **C 07 C 31/38**
C 07 C 29/14

(30) Priority: 29.12.83 GB 8334566

(43) Date of publication of application:
31.07.85 Bulletin 85/31

(84) Designated Contracting States:
BE CH DE FR GB IT LI NL

(71) Applicant: IMPERIAL CHEMICAL INDUSTRIES PLC
Imperial Chemical House Millbank
London SW1P 3JF(GB)

(72) Inventor: Bowden, Roy Dennis
Commonside School Lane
Norley Warrington WA6 6LG(GB)

(74) Representative: Stephenson, Kenneth et al,
Imperial Chemical Industries PLC Legal Department:
Patents PO Box 6
Welwyn Garden City Herts, AL7 1HD(GB)

(54) Method for the preparation of 2,2,2-trifluoroethanol.

(57) A method for the preparation of 2,2,2,-trifluoroethanol by contacting trifluoroacetaldehyde or its hydrate with hydrogen and a hydrogenation catalyst at an elevated temperature.

EP 0 149 905 A2

QM.32975

TITLE

CHEMICAL PROCESS          see front page

This invention relates to a chemical process and more particularly to a method for the preparation of 2,2,2-trifluoroethanol.

Trifluoroethanol is a known compound useful for a variety of purposes. Thus, in addition to being useful in the production of trifluoroethyl vinyl ether and 1-chloro-2,2,2-trifluoroethyl difluoromethyl ether, both anaesthetics, it has been proposed for use as a refrigerant, a heat pump working fluid and a solvent.

The methods that have been proposed for making trifluoroethanol have generally not been satisfactory for commercial operation because they involve the use of costly starting materials or intermediates. These methods include the hydrogenation of trifluoroacetic acid and its esters and the acetolysis of trifluoroethyl chloride followed by hydrolysis.

It has now been found that trifluoroethanol may readily be prepared by the hydrogenation of trifluoroacetaldehyde.

Accordingly, the invention provides a method for the preparation of 2,2,2-trifluoroethanol by contacting trifluoroacetaldehyde and hydrogen with a hydrogenation catalyst at an elevated temperature.

Hydrogenation catalysts which may be used in accordance with the invention include metals of Group VIII of the Periodic Table such as rhodium, rhenium, nickel, cobalt and, especially, ruthenium. The metals may be employed in elemental form or as compounds, for example metal oxides or salts or metal complexes.

The trifluoroacetaldehyde may be hydrogenated in either the gas or liquid phase. For a gas phase hydrogenation, the catalyst may be present as a metal or compound deposited on a solid support such as carbon, alumina, aluminium fluoride or calcium sulphate, ruthenium on carbon being particularly effective. A liquid phase hydrogenation may be performed in a suitable liquid medium, for example an aqueous medium optionally containing an organic solvent, using either a homogeneous or heterogeneous reaction system. In a homogeneous system, a catalytic metal in the form of a gauze or a suspended powder or a supported catalyst as already described for the gas phase reaction.

Suitable reaction temperatures are generally though not exclusively above 100°C, for example about 350°C, whilst the pressure can be atmospheric or higher. The reaction may be operated as a continuous or batch process and the trifluoroethanol product may be isolated and purified using conventional techniques. A convenient method of isolating the trifluoroethanol is to subject the reaction product to distillation, the desired product being collected in the distillate whilst any unchanged trifluoroacetaldehyde or its hydrate remains in the still residues.

The trifluoroacetaldehyde used in the method of the invention may be obtained in known manner, for example by reacting trichloroacetaldehyde with a stoichiometric excess of hydrogen fluoride at an elevated temperature, for example about 350°C, in the presence of a fluorination catalyst, for example chromia. It is particularly advantageous feature of the invention that trifluoroacetaldehyde obtained in this way may be hydrogenated in accordance with the invention without first being purified, dried or freed from acidic by-products. The trifluoroacetaldehyde may also be used in the form of the hydrate.

Thus, in a preferred embodiment of the invention, trichloroacetaldehyde, a readily available starting material, is perfluorinated by reaction with hydrogen fluoride and the resulting trifluoroacetaldehyde is, with optional neutralisation, catalytically hydrogenated to give the desired trifluoroethanol.

The invention is illustrated but not limited by the following Examples:

Example 1

Technical grade chloral (0.6 mls/min) was vapourised and passed into the bottom of a vertical 2.5 cm diameter Inconel reactor where it was mixed with nitrogen (40 mls/min) and gaseous hydrogen fluoride (500 mls/min). The reactor tube contained a 43 cm bed length of Chromia catalyst which was held at approx 370°C. The contact time was 6 secs.

The off gases from this reactor were mixed with hydrogen (1750 mls/min) and passed over a second horizontal catalyst bed in a 2.5 cm Inconel reactor tube. This reactor contained a 68 cm bed length of 1% ruthenium on Sutcliffe Speakman 5-10 mesh grade 610 carbon, pretreated with hydrogen and held at 250°C. The contact time was calculated as 4 secs.

The off gases from the second reactor were passed into water and the organic content analysed by Gas Liquid Chromatography either directly or after neutralisation using either Chromosorb 101 at 130 (or 180°C) or Poropak Q at 180°C. This showed that the organic content of the mixture comprised fluoral 54% and trifluoroethanol 37%. The trifluoroethanol was confirmed by $F^{19}$ NMR spectroscopy.

Examples 8-22

Into the bottom of a vertical 2.5 cm diameter Inconel reactor tube containing chromia were passed hydrogen fluoride (893 ml/min, measured as liquid). The catalyst had a bed length of 43 cm and was held at 370°C. The contact time was 5 secs.

The off gases were passed into water and the pH was adjusted by addition of KOH solution. This mixture was used for subsequent reductions which were carried out in a 1 litre electrically heated Baskerville Inconel autoclave stirred at 1000 rpm and fitted with a pressure gauge. Hydrogen at the stated pressure was applied.

Other gas phase examples Run under essentially the same conditions as example 1.

| | REACTOR 1 | | | | | | | REACTOR 2 | | | | | ANALYSIS |
|---|---|---|---|---|---|---|---|---|---|---|---|---|---|
| | CATALYST | BED LENGTH | CHLORAL FEED | HF FEED | $N_2$ FEED | TEMP °C | CONTACT TIME | CATALYST | BED LENGTH | $H_2$ FEED | TEMP °C | CONTACT TIME | |
| 2 | Chromia | 43 | 0.38 | 500 | 40 | 370 | 9 | 1% Ru on C | 17 | 340 | 250 | 2.7 | Fluoral 99% TFEA 1% |
| 3 | -"- | 43 | 0.34 | 420 | 40 | 370 | 10 | 1% Ru on C | 68 | 1680 | 325 | 4.3 | Fluoral TFEA 24% |
| 4 | -"- | 43 | 0.34 | 420 | 40 | 370 | 10 | 4% Ru on C | 68 | 1680 | 325 | 4.3 | Fluoral 31% TFEA 64% |
| 5 | -"- | 39 | 0.64 | 790 | 40 | 400 | 5 | 1% Pt on C | 28 | 190 | 140 | 4.9 | No alcohol seen |
| 6 | -"- | 38 | 0.75 | 735 | 40 | 400 | 4.9 | 1%Pd on $AL_2O_3$ | 31 | 220 | 120 | 5.5 | -"- |
| 7 | -"- | 39 | 0.64 | 790 | 40 | 400 | 5 | Cu/Cr | 39 | 190 | 300 | 4.9 | -"- |

0149905

EXAMPLES 8-22

| | CATALYST | WEIGHT | TEMP | PRESSURE | | TIME | % ORGANICS AS TFEA |
|---|---|---|---|---|---|---|---|
| 8 | 4% Ru on C | 2g | 200°C | 20 | bars | 3.5 | 84% |
| 9 | – " – | –"– | 200 | 20 | " | 1.5 | 81% |
| 10 | – " – | –"– | 100 | 17 | " | 21.0 | 25% |
| 11 | – " – | –"– | 150 | 20 | " | 5.0 | 76% |
| 12 | – " – | –"– | 150 | 20 | " | 21.0 | 96% |
| 13 | 5% Ru on C | –"– | 220 | 20 | " | 0.5 | 97% |
| 14 | Ni/Kieselguhr | –"– | 220 | 20 | " | 3.0 | 98% |
| 15 | – " – | –"– | 220 | 20 | " | 2.0 | 83% |
| 16 | – " – | –"– | 150 | 10 | " | 3.0 | 98% |
| 17 | – " – | 0.5g | " | 10 | " | 3.0 | 99% |
| 18 | – " – | 0.1g | " | 10 | " | 3.4 | 99% |
| 19 | 4% Ru on C | 0.5g | " | 10 | " | 3.0 | 99% |
| 20 | Pt | 0.2g | " | 10 | " | 15.0 | 98% |
| 21 | Pd | 0.2g | " | 10 | " | 3.0 | 95% |
| 22 | Rh | 0.25g | " | 10 | " | 1.5 | 95% |

0149905

1.    A method for the preparation of 2,2,2-trifluoroethanol by contacting trifluoroacetaldehyde or its hydrate with hydrogen and a hydrogenation catalyst at an elevated temperature.

2.    A method according to claim 1 wherein trichloroacetaldehyde is perfluorinated by reaction with hydrogen fluoride and the resulting trifluoroacetaldehyde is passed to a second reaction zone where it is hydrogenated.